# EUROPÄISCHE PATENTSCHRIFT

(11) **EP 2 538 838 B1**
(45) Veröffentlichungstag und Bekanntmachung des Hinweises auf die Patenterteilung: **30.11.2016**
(21) Anmeldenummer: 11708754.4
(22) Anmeldetag: 23.02.2011
(51) Int. Cl.: A61B 5/0408, A61B 5/042, A61B 5/0492, A61N 1/05, A61B 5/00, A61B 5/04

(54) **HYBRIDES DREIDIMENSIONALES SENSORARRAY, INSBESONDERE ZUR VERMESSUNG ELEKTROGENER ZELLANORDNUNGEN, SOWIE MESSANORDNUNG**
HYBRID THREE-DIMENSIONAL SENSOR ARRAY, IN PARTICULAR FOR MEASURING ELECTROGENIC CELL ASSEMBLIES, AND MEASURING ASSEMBLY
BARRETTE DE CAPTEURS HYBRIDE, TRIDIMENSIONNELLE, EN PARTICULIER POUR LA MESURE D'AGENCEMENT DE CELLULES ÉLECTROGÈNES, AINSI QUE DISPOSITIF DE MESURE

(30) Priorität: 26.02.2010 DE 102010000565
(43) Veröffentlichungstag der Anmeldung: 02.01.2013
(73) Patentinhaber: Technische Universität Ilmenau, 98693 Ilmenau (DE)
(72) Erfinder: SCHOBER, Andreas, 90762 Fürth (DE); HAMPL, Jörg, 99087 Erfurt (DE); FERNEKORN, Uta, 99094 Erfurt (DE); HUSAR, Peter, 98693 Ilmenau (DE); FISCHER, Michael, 07407 Uhlstädt-Kirchhasel (DE); LAQUA, Daniel, 98693 Ilmenau (DE); LILIENTHAL, Katharina, 01277 Dresden (DE)
(74) Vertreter: Engel, Christoph Klaus
(86) Internationale Anmeldenummer: PCT/EP2011/052638
(87) Internationale Veröffentlichungsnummer: WO 2011/104250

(56) Entgegenhaltungen:
- WO-A1-2010/005479
- JP-A- 2004 237 077
- US-A1- 2010 029 148

## Beschreibung

Die vorliegende Erfindung betrifft ein dreidimensionales Sensorarray, welches sich insbesondere zur Aufnahme elektrischer Signale eignet, die in natürlichen Zellverbindungen auftreten. Bei den zu vermessenden Zellanordnungen handelt es sich beispielsweise um Gewebeabschnitte im tierischen oder menschlichen Organismus. Speziell ermöglicht die Erfindung die Aufnahme elektrischer oder elektromagnetischer Signale, die von Neuronen erzeugt und an umliegende Neuronen oder muskuläre Zellen weitergeleitet werden. Ebenso findet das erfindungsgemäße Sensorarray Einsatz bei der Untersuchung von Zellkulturen, die außerhalb eines Organismus gezüchtet werden, beispielsweise in einem Kultursystem.

Zur Erfassung von in biologischem Gewebe auftretenden elektrischen Signalen wurden in der Vergangenheit zwei grundsätzlich verschiedene Lösungsansätze verfolgt. Seit langer Zeit ist es möglich, mit flächig angebrachten Elektroden, beispielsweise auf der Hautoberfläche eines Patienten bei der Aufnahme eines EEG, ein Summensignal aufzuzeichnen, wie es an der Oberfläche eines biologischen Gewebes auftritt. Die genaue Position der Entstehung und Weitergabe derartiger Signale innerhalb des biologischen Gewebes lässt sich mit dieser Methode nicht untersuchen. In jüngerer Zeit versucht man, die im biologischen Gewebe erzeugten Signale und die dort ablaufenden Prozesse der biologischen Ionenleitung näher zu untersuchen, indem an einzelnen Positionen innerhalb eines dreidimensionalen Gewebekörpers Messelektroden positioniert werden, um die Signale punktuell aufnehmen zu können. Problematisch ist dabei allerdings, dass der genaue Entstehungsort der Signale und der Weg ihrer Weiterleitung nicht bekannt sind, sodass die Positionierung der Elektroden sehr schwierig ist. Die Signalverteilung im Raum kann mit solchen Sonden ebenfalls nicht bestimmt werden. Grundsätzlich besteht bei der Signalerfassung innerhalb von biologischem Gewebe weiterhin das Problem, dass es aufgrund der sich aufbauenden elektrochemischen Spannungsreihe zu einer Korrosion der Elektroden und/oder mittelfristig zu einer Gewebeveränderung kommt, wodurch die erfassten Signale verfälscht werden. Dieselbe Problematik besteht, wenn zu Stimulationszwecken elektrische Signale über die Elektroden in das biologische Gewebe eingespeist werden sollen.

In jüngerer Zeit wurden Sensoren vorgeschlagen, die das Problem der exakten Positionierung der Elektrode innerhalb des Gewebes entschärfen sollen. Beispielsweise wurde das sogenannte Utah Electrode Array beschrieben, bei welchem es sich um ein miniaturisiertes Sensorarray handelt, welches auf einem Träger zahlreiche Sensornadeln besitzt, die jeweils an ihrer Sensorspitze eine Elektrode aufweisen. Um die Signalerfassung in unterschiedlich tiefen Schichten des Gewebes zu ermöglichen (Z-Richtung), können die Sensornadeln unterschiedliche Längen aufweisen, sodass sie beim Eindringen in das Gewebe unterschiedlich tief in dieses eindringen ("Utah Electrode Array to Control Bionic Arm"; 24.05.2006; http://www.medgadget.com/archives/print/002076print.html). Aber auch mit diesem Sensorarray lässt sich die räumliche Verteilung elektrischer Signale in biologischem Gewebe nur sehr beschränkt erfassen, denn jede Sensornadel des Arrays erfasst Signale nur in einer bestimmten Tiefe im Gewebe. Außerdem besteht aufgrund der Konstruktion des Sensorarrays das Problem, dass eine ungehinderte Fluidströmung durch das Array von der durchgehenden Trägerplatte verhindert wird, wodurch die Nährstoffversorgung von Zellkulturen in Kultursystemen erheblich beeinträchtigt ist.

Aus der JP 2004237077 A ist ein dreidimensionales Sensorarray mit kammartig angeordneten und gegenseitig in x- und y-Richtung beabstandeten Sensornadeln bekannt. Jede Sensornadel besitzt mehrere in Längsrichtung auf der Sensornadel verteilte Elektrodenflächen.

Die US 2003/0100823 A1 zeigt ein dreidimensionales Sensorarray mit mehreren kammartig angeordneten Sensornadeln. Jede Sensornadel ist mit mehreren in Längsrichtung auf der Sensornadel verteilt angeordneten Elektrodenflächen versehen.

Die WO 2010/005479 A1 beschreibt ein dreidimensionales Sensorarray zur Vermessung elektrischer Signale in biologischen Zellanordnungen. Bei dem aus dieser Druckschrift vorbekannten Sensorarray weist jede Sensornadel nur eine Elektrodenfläche auf.

Die US 2010/0029148 A1 zeigt ein Mikrosystem mit einem Siliziumsubstrat, in welches voneinander beabstandete Schlitze eingebracht sind, wobei die Schlitze derart geformt sind, dass sie im Inneren des Substrats einen Vorsprung aufweisen. Die Schlitze dienen der Aufnahme der hinteren Enden mikrostrukturierter Sensorplatten, wobei diese hinteren Enden mittels Vorsprung gehaltert werden. An den hinteren Enden sind mehrere Sensornadeln kammartig angeordnet, welche sich durch das Substrat nach außen erstrecken. Durch die Anordnung der Sensorplatten in voneinander beabstandeten Schlitzen sind auch hier die Trägerabschnitte als auch die Sensornadeln benachbarter Sensorplatten voneinander in einer zweiten Richtung beabstandet. Im Bereich zwischen den Sensornadeln befindet sich kein Substrat, wodurch auch eine Fluidströmung zwischen den Sensornadeln in Längsrichtung möglich ist.

Eine Aufgabe der vorliegenden Erfindung besteht somit darin, ein verbessertes dreidimensionales Sensorarray bereitzustellen, mit welchem elektrische Signale in einem dreidimensionalen biologischen Zellverbund präzise erfasst werden können, insbesondere in Bezug auf Zeit und Ort des Auftretens solcher Signale. Eine Teilaufgabe wird darin gesehen, ein Sensorarray derart zu modifizieren, dass eine stromlose Vermessung in Gewebestrukturen möglich wird, um die Korrosion von Elektroden und Gewebeveränderungen zu verhindern. Schließlich besteht eine andere Teilaufgabe darin, das Sensorarray derart zu modifizieren, dass es sich nicht nur für den Einsatz im lebenden Organismus sondern insbesondere auch für die Vermessung von im Bioreaktor kultivierten Zellverbünden eignet und die Nährstoffversorgung der kultivierten Zellen nicht beeinträchtigt.

Die zuvor genannte Hauptaufgabe wird von einem dreidimensionalen Sensorarray gemäß dem beigefügten Patentanspruch 1 erfüllt. Die genannten Teilaufgaben werden insbesondere durch bevorzugte Ausführungsformen gemäß den Unteransprüchen erfüllt.

Das erfindungsgemäße Sensorarray ist aus mehreren mikrostrukturierten Sensorplatten zusammengesetzt, die je einen Trägerabschnitt aufweisen, an welchem jeweils mehrere Sensornadeln kammartig angeordnet sind. Die Sensornadeln sind in einer ersten Richtung (X-Richtung) voneinander beabstandet und tragen jeweils mehrere Elektrodenflächen, die in Längsrichtung der Sensornadel (Z-Richtung) verteilt sind. Jede der Elektrodenflächen ist über eine eigene Leiterbahn kontaktiert, wobei alle Leiterbahnen über den Trägerabschnitt zu einem Kontaktierungsabschnitt verlaufen. Zwischen den mehreren Sensorplatten befinden sich Abstandselemente, die der Beabstandung der Sensorplatten und vorzugsweise gleichzeitig der Befestigung dieser Platten dienen. Auf diese Weise sind die Trägerabschnitte und die daran ausgebildeten Sensornadeln jeweils von den benachbarten Sensorplatten in einer zweiten Richtung (Y-Richtung), die senkrecht zur ersten Richtung und zur Längsrichtung der Sensornadeln (Z) verläuft, voneinander beabstandet. Zwischen den Abstandselementen und den Trägerabschnitten sind Durchlässe ausgebildet, die eine durch das Sensorarray verlaufende Fluidströmung zwischen den Sensorplatten in Längsrichtung der Sensornadeln gestatten.

Durch den erfindungsgemäßen Aufbau des Sensorarrays sind zahlreiche Elektrodenflächen ausgebildet, die im Raum gitterartig verteilt angeordnet sind. Wenn das Sensorarray in einem biologischen Gewebe eingebracht ist, lassen sich in dem Raum, in dem sich die Sensornadeln erstrecken, auftretende elektrische Signale hinsichtlich des Orts genau bestimmen. Da sämtliche Elektrodenflächen einzeln kontaktiert sind und damit die jeweils erfassten Signale an eine Auswerteeinheit weitergeleitet werden können, lässt sich die entstehende Signalmenge zeitlich und örtlich auflösen, sodass sowohl der Entstehungspunkt als auch die Arte der Weiterleitung von Signalen im Gewebeverbund aufgezeichnet werden können.

Die Sensornadeln im Sensorarray können als Nadelstrukturen vorzugsweise aus Silizium oder glasartigen Siliziumdioxidoberflächen mit metallischem Kern durch an sich bekannte Verfahren der Nanotechnologie hergestellt werden. Beispielsweise lassen sich selbstorganisierende Ätz-, Aufwachs- und Formprozesse anwenden. Dabei ist es auch möglich, an den Sensornadeln geometrische Oberflächenstrukturen auszubilden, die eine Verankerung in biologischem Gewebe erleichtern. Mikrobauteile mit derart ausgeformten nanostrukturierten Oberflächen sind beispielsweise aus der WO 2007/017458 A1 bekannt, auf welche hinsichtlich der Erzeugung solcher Oberflächenstrukturen verwiesen wird.

Gemäß einer bevorzugten Ausführungsform der vorliegenden Erfindung erstrecken sich die Abstandselemente ausschließlich zwischen den Trägerabschnitten der Sensorplatten, sodass zwischen den Sensornadeln benachbarter Sensorplatten Freiräume verbleiben, die durch das zu untersuchende biologische Gewebe ausgefüllt werden können. Durch die zwischen den Abstandselementen und den Trägerabschnitten ausgebildeten Durchlässe wird eine Fluidströmung durch das Sensorarray in Z-Richtung ermöglicht. Das Sensorarray kann damit sehr einfach als Bestandteil eines Kultursystems gestaltet werden, wobei die Nährstoffzufuhr zu den einzelnen Gewebeschichten durch die Positionierung der Sensorarrays nicht beeinträchtigt oder sogar erleichtert wird.

Vorteilhaft ist die essentielle Sensitivitätserhöhung der elektrischen Messung durch die nadelartigen, grasartigen Nanostrukturen an der Oberfläche der Sensornadeln. Gleichzeitig können diese Nanostrukturen auf der Fügefläche zur nächsten Sensorplatte angebracht sein und so zur neuartigen Aufbau- und Verbindungstechnik zum realen 3D-MEA beitragen, indem sie in den abstandshaltenden Kunststoff eingepresst werden. Solche neuartigen Aufbau- und Verbindungstechniken, angewandt auf zusätzlich kapazitiv wirksame Materialien, machen die Dreidimensionalität der beschriebenen Sensoren möglich.

Die Oberfläche der Sensornadeln ist biologisch passiviert. Durch Anbringung entsprechender Beschichtungen kann die Entstehung elektrochemischer Spannungsreihen verhindert werden. Grundsätzlich ist die Vorgehensweise für eine biologische Passivierung von Halbleitermaterialien, wie sie für die Erzeugung der Sensornadeln verwendbar sind, dem Fachmann bekannt, sodass auf eine detaillierte Beschreibung verzichtet wird. In diesem Zusammenhang ist es jedoch besonders vorteilhaft, wenn auch die Elektrodenflächen mit einer elektrisch isolierenden, insbesondere biologisch passivierten Abdeckung beschichtet sind. Die Signalerfassung erfolgt in diesem Fall durch kapazitive Messmethoden, wobei die einzelnen Elektrodenflächen jeweils eine Elektrode eines Messkondensators bilden. Die benötigte Gegenelektrode kann durch gegenüberliegende Elektrodenflächen an den Sensornadeln oder auch durch eine gemeinsame Kondensatorplatte realisiert sein, welchen einen eigenständigen Bestandteil des Sensorarrays darstellt. Um das Übersprechen bei der Signalerfassung zu verringern, können die Leiterbahnen im Sensorarray mit einer elektromagnetisch wirksamen Schirmung versehen werden.

Die oben genannte Aufgabe wird erfindungsgemäß auch durch eine Messanordnung gemäß dem nebengeordneten Anspruch 5 gelöst. Diese Messanordnung umfasst ein zuvor beschriebenes Sensorarray sowie eine daran angeschlossene Auswerteeinheit, welche die von den mehreren Elektrodenflächen des Sensorarrays gelieferten Signale zeitlich und örtlich erfasst und verarbeitet. Die Auswerteeinheit oder Teile davon können als On-Chip-Signalverarbeitungsschaltung aufgebaut sein und in unmittelbarer Nähe der Elektrodenflächen am Sensorarray angeordnet werden. Dadurch lässt sich eine Datenreduktion On-Chip ausführen, sodass eine reduzierte Datenmenge beispielsweise durch eine drahtlose Kommunikationsverbindung an eine externe Datenverarbeitungseinheit übertragen werden kann. Vorzugsweise umfasst die Messanordnung darüber hinaus einen Signalgenerator, der ein elektrisches Stimulationssignal an eine oder mehrere Elektrodenflächen des Sensorarrays liefern kann. Damit können im biologischen Gewebe nicht nur die dort natürlich erzeugten Signale erfasst werden, sondern es ist eine gezielte Stimulation möglich, beispielsweise um Muskelzellen zu aktivieren oder andere Prozesse im Gewebeverbund zu simulieren.

Weitere Vorteile, Einzelheiten und Weiterbildungen der vorliegenden Erfindung ergeben sich aus der nachfolgenden Beschreibung bevorzugter Ausführungsformen, unter Bezugnahme auf die Zeichnung. Es zeigen:
- Fig. 1:: eine vereinfachte Darstellung einer Sensorplatte mit mehreren Sensornadeln in einer Draufsicht;
- Fig. 2:: eine Anordnung mehrerer Sensorplatten auf einem Wafer während eines Herstellungsschrittes;
- Fig. 3:: eine perspektivische Darstellung eines Abstandselements;
- Fig. 4:: eine perspektivische Darstellung einer ersten Ausführungsform eines dreidimensionalen Sensorarrays;
- Fig. 5:: eine Zusammenbauzeichnung mit abgewandelten Ausführungsformen der Bestandteile des Sensorarrays;
- Fig. 6:: eine perspektivische Darstellung eines Zellkultivierungssystems mit integriertem Sensorarray.

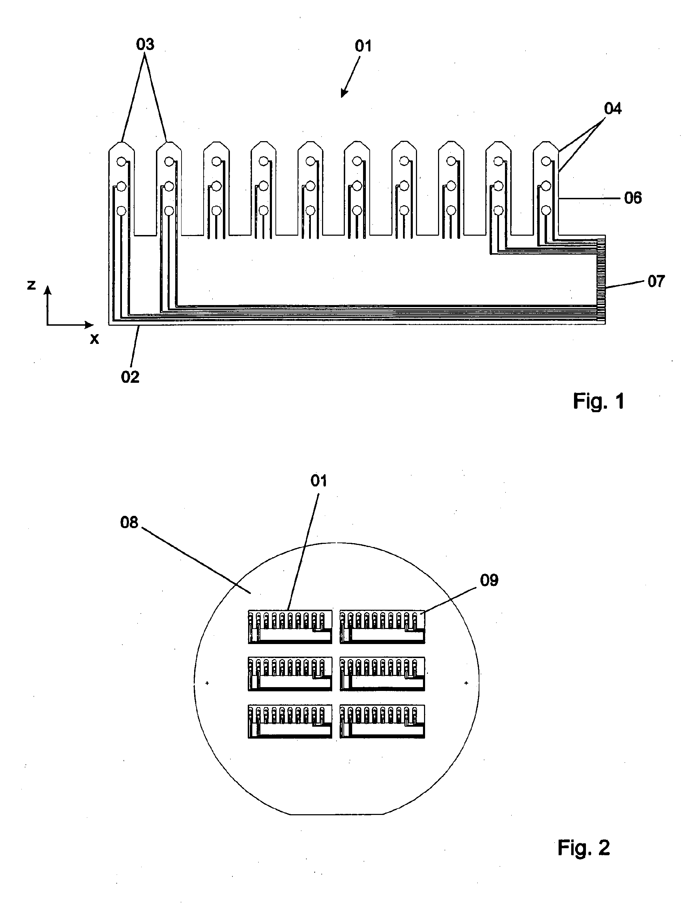

In Fig. 1 ist ein erster Bestandteil des erfindungsgemäßen Sensorarrays in einer Draufsicht vereinfacht dargestellt. Es handelt sich dabei um eine Sensorplatte 01, die durch Mikrostrukturierung hergestellt ist und einen Trägerabschnitt 02 sowie zahlreiche Sensornadeln 03 besitzt. Die Sensornadeln 03 sind kammartig am Trägerabschnitt 02 angeordnet und voneinander in X-Richtung beabstandet. Der Abstand zwischen den einzelnen Sensornadeln beträgt beispielsweise 50 bis 1000 µm. An jeder Sensornadel 03 sind mehrere Elektrodenflächen 04 angeordnet, die in Z-Richtung (Längsrichtung der Sensornadel) voneinander beabstandet sind. Jede Elektrodenfläche ist an eine eigene Leiterbahn 06 angeschlossen, sodass auf der Sensorplatte zahlreiche Leiterbahnen 06 verlaufen, die über den Trägerabschnitt 02 zu einem Kontaktierungsabschnitt 07 geführt sind.

Fig. 2 zeigt die Anordnung mehrerer Sensorplatten 01 auf einem Wafer 08 während eines Herstellungsschrittes. In dieser Phase der Herstellung sind die Sensornadeln 03 zunächst noch von einem Strukturierungsbereich 09 umgeben, der später z.B. durch Ätzen oder Sandstrahlen entfernt werden muss, um die kammartige Struktur der Sensornadeln freizulegen. Die zunächst zweidimensionale Erzeugung der Strukturen auf den einzelnen Sensorplatten erfolgt vorzugsweise mittels Standard-MEMS-Technologien. Beispielsweise wird ein isolierendes Substrat (Glas, Borofloat 33) in Waferform als Ausgangsmaterial verwendet. Mithilfe von Dünnschichttechnologien (Sputtern, Bedampfen) werden Metallschichten abgeschieden, die nachfolgend durch Lithografie und Ätzen strukturiert werden können. Um die Beeinflussung der später zu untersuchenden Zellkulturen durch das Sensorarray gering zu halten, wird eine isolierende, biokompatible Passivierungsschicht (vorzugsweise Si₃N₄ oder SiO₂) mit einem Niedertemperatur-Abscheideverfahren (PECVD) über der gesamten Struktur abgeschieden. Durch einen weiteren Ätzschritt werden die Elektrodenflächen 04 nachfolgend wieder freigelegt, soweit nicht eine kapazitive Messung bevorzugt wird. Entsprechende Strukturierungsschritte können auf beiden Seiten der Waferscheibe ausgeführt werden, um Elektrodenflächen beidseitig an den Sensornadeln anzubringen. Abweichende Herstellungsschritte sind erforderlich, wenn die Leiterbahnen 06 zusätzlich mit einer Schirmung versehen werden sollen.

Nachdem die Elektrodenflächen und die Leiterbahnen erzeugt wurden, muss die Kammstruktur für die einzelnen Sensornadeln hergestellt werden, wofür eine Strukturierung durch den kompletten Wafer erforderlich ist. Dazu können nass- und trockenchemische Ätzprozesse eingesetzt werden. Ebenso ist bei Verwendung von vorstrukturierten Masken ein Mikrosandstrahlen möglich, womit die Bearbeitungszeit drastisch reduziert wird. Die auf diese Weise hergestellten Sensorplatten werden durch Wafersägen nachfolgend vereinzelt, sodass mehrere Sensorplatten vorliegen.

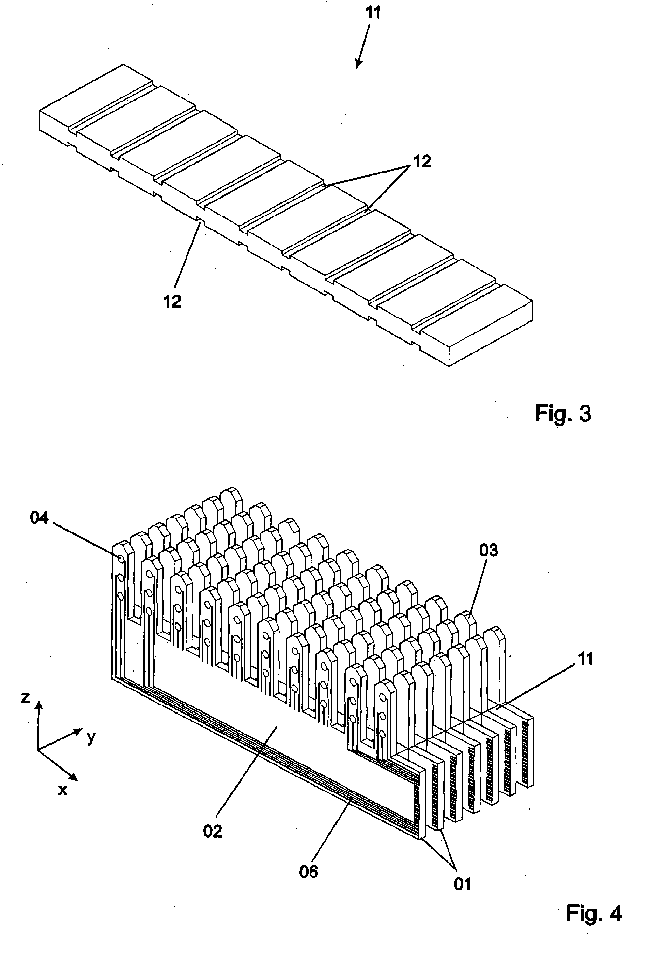

Fig. 3 zeigt in einer perspektivischen Ansicht ein Abstandselement 11, welches einen weiteren Bestandteil des erfindungsgemäßen Sensorarrays bildet. Das Abstandselement 11 besteht vorzugsweise aus Kunststoff, insbesondere Polycarbonat. In seinen Abmessungen entspricht das Abstandselement bezüglich Breite und Länge in etwa der Bemessung des Trägerabschnitts 02 der Sensorplatte. Die Dicke des Abstandselements bestimmt den späteren Abstand der einzelnen Sensorplatten in Y-Richtung und beträgt beispielsweise 50 bis 1000 µm. In dem Abstandselement 11 sind beidseitig mehrere Durchlässe 12 als nutförmige Vertiefungen ausgebildet. Im zusammengebauten Sensorarray bewirken diese Durchlässe 12, dass ein Fluidstrom, beispielsweise eine Nährstofflösung, durchströmen kann und somit zwischen den einzelnen Sensorplatten aufrechterhalten wird.

Fig. 4 zeigt eine perspektivische Ansicht einer ersten Ausführungsform des Sensorarrays. Dieses besteht ersichtlich aus mehreren Sensorplatten 01, die durch zwischengelegte Abstandselemente 11 in Y-Richtung voneinander beabstandet sind, sodass zahlreiche Sensornadeln 03 matrixartig angeordnet sind. Die auf den Sensornadeln 03 angebrachten Elektrodenflächen 04 sind über den durch die Sensornadeln definierten Raum verteilt. Der hybride dreidimensionale Aufbau des Sensorarrays erfolgt vorzugsweise durch Thermokompressionsbonden. Dazu werden die Abstandselemente 11 mit den Sensorplatten 01 alternierend gestapelt, in einer Thermo-Presse auf etwa 90 % der Erweichungstemperatur des Materials der Abstandselemente erhitzt und mit einem Druck von beispielsweise von 5 MPa beaufschlagt.

Die in Kontakt stehenden Oberflächen der Abstandselemente und der Sensorplatten können zuvor durch eine Plasma-Aktivierung vorbehandelt werden. Die erforderliche Thermo-Bond-Zeit beträgt etwa 3 min.

Wenn bei alternativen Ausführungsformen die Abstandselemente nicht aus Kunststoff sondern aus Silizium bestehen, kann die Verbindung zwischen den Abstandselementen und den Sensorplatten durch anodisches Bonden hergestellt werden. In diesem Fall muss der Stapel aus Abstandselementen und Sensorplatten sequenziell gebondet werden.

Es ist ersichtlich, dass durch den erfindungsgemäßen Aufbau ausreichend Raum zwischen den Sensornadeln 03 verbleibt, damit sich dort biologische Zellen ansiedeln können. Das Sensorarray lässt sich in natürliche Zellumgebungen einbringen, indem die Sensornadeln in das Gewebe geschoben werden. Anders als bei anderen matrixartigen Sensorarrays bleibt eine Fluidströmung auch in Z-Richtung möglich, da trotz der erforderlichen Ableitung der zahlreichen Leiterbahnen auf den Trägerabschnitten zwischen den einzelnen Sensorplatten Strömungskanäle mithilfe der Durchlässe 12 gebildet sind. Eine derartige Durchströmung ist insbesondere bei der Kultivierung von biologischen Zellen erforderlich, um ausreichend Nährstofflösung an sämtliche Zellen in einem dreidimensionalen Verbund heranzuführen.

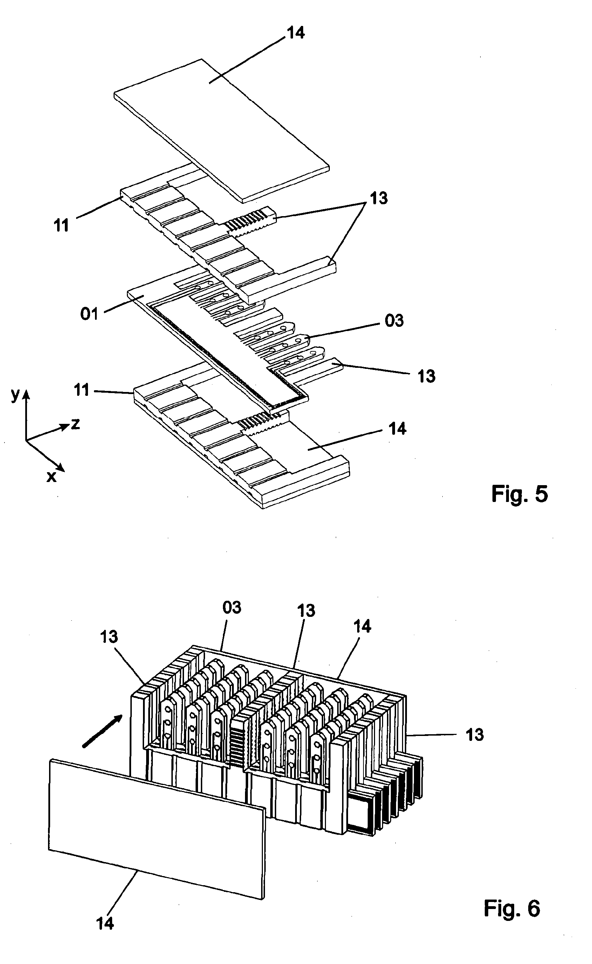

Fig. 5 zeigt eine abgewandelte Ausführungsform der Bestandteile des Sensorarrays in einer Zusammenbauzeichnung. Sowohl die Sensorplatten 01 als auch die Abstandselemente 11 besitzen bei dieser Ausführungsform Trennstege 13, die in Z-Richtung etwa die Länge der Sensornadeln 03 besitzen. In X-Richtung sind die Trennstege 13 gleichmäßig positioniert, sodass sie nach dem Zusammenbau des Plattenstapels an den jeweiligen Trennstegen der benachbarten Platten (Sensorplatte bzw. Abstandselement) dicht anliegen. Weiterhin sind an den Rändern des Plattenstapels zusätzliche Abdeckplatten 14 vorgesehen, die den Raum der dazwischenliegenden Sensornadeln einschließen.

Fig. 6 zeigt in einer perspektivischen Ansicht den weitgehend zusammengebauten Zustand einer abgewandelten Ausführungsform des Sensorarrays, der in diesem Fall integraler Bestandteil eines Zellkultivierungssystems ist. Durch die äußeren Trennstege 13 sowie die Abdeckplatten 14 wird ein Kultivierungsraum erzeugt, in welchem mehrere Sensornadeln 03 angeordnet sind, wobei zwischen diesen eine Zellkultur gezüchtet werden kann. Bei der dargestellten Ausführungsform ist der Kultivierungsraum in zwei durch mittlere Trennstege 13 getrennte Kammern unterteilt. Zwischen den beiden Kammern kann über in den mittleren Trennstegen vorgesehene Kanäle eine Kommunikation erfolgen, sodass Fluide strömen können und/oder eine Zellemigration stattfinden kann. Beispielsweise können in einer Kammer Neuronen kultiviert werden, während in der anderen Kammer Muskelzellen wachsen. Axone der Neuronen können durch die Kanäle in den mittleren Trennstegen hindurchwachsen und an die Muskelzellen andocken. Mithilfe des Sensorarrays lassen sich in beiden Kammern die entstehenden Signale und deren Ausbreitung örtlich und zeitlich aufgelöst bestimmen.

### Bezugszeichenliste

- 01 -: Sensorplatte
- 02 -: Trägerabschnitt
- 03 -: Sensornadel
- 04 -: Elektrodenflächen
- 05 -: -
- 06 -: Leiterbahn
- 07 -: Kontaktierungsabschnitt
- 08 -: Wafer
- 09 -: Strukturierungsbereich
- 10 -: -
- 11 -: Abstandselement
- 12 -: Durchlässe
- 13 -: Trennstege
- 14 -: Abdeckplatte

## Patentansprüche

1. Dreidimensionales Sensorarray zur Vermessung elektrischer Signale in biologischen Zellanordnungen, umfassend:
- mehrere mikrostrukturierte Sensorplatten (1), mit je einem Trägerabschnitt (2), an welchem mehrere Sensornadeln (3) kammartig angeordnet sind, sodass sie in einer ersten Richtung (X) voneinander beabstandet sind, wobei jede Sensornadel (3) mehrere in Längsrichtung (Z) auf der Sensornadel (3) verteilte Elektrodenflächen (4) besitzt, die jeweils an eine eigene Leiterbahn (6) kontaktiert sind, und wobei die Leiterbahnen (6) über den Trägerabschnitt (2) zu einem Kontaktierungsabschnitt (7) verlaufen;
- mehrere Abstandselemente (11), die zwischen den Sensorplatten (1) befestigt sind, sodass sowohl die Trägerabschnitte (2) als auch die Sensornadeln (3) jeweils benachbarter Sensorplatten (1) voneinander in einer zweiten Richtung (Y) beabstandet sind,
**dadurch gekennzeichnet, dass** zwischen den Abstandselementen (11) und den Trägerabschnitten (2) Durchlässe (12) ausgebildet sind, die eine durch das Sensorarray verlaufende Fluidströmung zwischen den Sensorplatten (1) in Längsrichtung (Z) der Sensornadeln (3) gestatten, dass die Sensorplatten aus einem isolierendem Substrat bestehen, und dass die Oberfläche der Sensorplatten eine isolierende, biokompatible Passivierungsschicht aufweist.

2. Sensorarray nach Anspruch 1, **dadurch gekennzeichnet, dass** sich die Abstandselemente (11) ausschließlich zwischen den Trägerabschnitten (2) der Sensorplatten (1) erstrecken und zwischen den Sensornadeln (3) Freiräume belassen.

3. Sensorarray nach Anspruch 1 oder 2, **dadurch gekennzeichnet, dass** die Leiterbahnen (6) mit einer elektromagnetisch wirksamen Schirmung versehen sind.

4. Sensorarray nach einem der Ansprüche 1 bis 3, **dadurch gekennzeichnet, dass** die Sensornadeln (3) an ihren Oberflächen widerhakenförmige Nanostrukturen aufweisen.

5. Messanordnung zur Vermessung elektrischer Aktivitäten biologischer Zellanordnungen, **dadurch gekennzeichnet, dass** sie ein Sensorarray nach einem der Ansprüche 1 bis 4 umfasst, welches an eine Auswerteeinheit angeschlossen ist, welche die von den mehreren Elektrodenflächen (4) des Sensorarrays gelieferten Signale zeitlich und örtlich aufgelöst erfasst und verarbeitet.

6. Messanordnung nach Anspruch 5, **dadurch gekennzeichnet, dass** die Auswerteeinheit Kapazitätsänderungen an den Elektrodenflächen (4) erfasst und auswertet, wobei die einzelnen Elektrodenflächen (4) jeweils eine Elektrode eines Messkondensators bilden, wobei die Gegenelektrode des Messkondensators jeweils durch eine gegenüberliegende Elektrodenfläche (4) an einer Sensornadel (3) oder eine gemeinsame Kondensatorplatte gebildet ist.

7. Messanordnung nach Anspruch 5 oder 6, **dadurch gekennzeichnet, dass** sie einen Signalgenerator umfasst, der bei Aktivierung ein elektrisches Stimulationssignal an eine oder mehrere der Elektrodenflächen (4) liefert.

## Claims

1. A three-dimensional sensor array for measuring electrical signals in biological cell assemblies, comprising:
- several micro-structured sensor plates (1), each with a carrier section (2), on which several sensor needles (3) are arranged in a comb-like manner, so that they are spaced from each other in a first direction (X), whereby each sensor needle (3) comprises several electrode surfaces (4) distributed in the longitudinal direction (Z) on the sensor needle (3) which electrode surfaces are contacted on their own conducting track (6), and whereby the conducting tracks (6) run via the carrier section (2) to a contacting section (7);
- several spacing elements (11) fastened between the sensor plates (1), so that the carrier sections (2) as well as the sensor needles (3) of adjacent sensor plates (1) are spaced from each other in a second direction (Y),
**characterized in that** passages (12) are formed between the spacer elements (11) and the carrier sections (2), which passages allow a flow of fluid running through the sensor array between the sensor plates (1) in the longitudinal direction (Z) of the sensor needles (3), **in that** the sensor plates consist of an insulating substrate, and **in that** the surface of the sensor plates having an insulating, biologically compatible passivated covering.

2. Sensor array according to claim 1, **characterized in that** the spacer elements (11) extend exclusively between the carrier sections (2) of the sensor plates (1) and leave free spaces between the sensor needles (3).

3. Sensor array according to claim 1 or 2, **characterized in that** the conducting tracks (6) are provided with an electromagnetically active screening.

4. Sensor array according to one of claims 1 to 3, **characterized in that** the sensor needles (3) comprise barbed nanostructures on their surfaces.

5. Measuring assembly for measuring electrical activities of biological cell assemblies, **characterized in that** it comprises a sensor array according to one of claims 1 to 4, which is connected to an evaluation unit that detects and processes in time and as to location in a resolved manner the signals delivered from the several electrode surfaces (4) of the sensor array.

6. Measuring assembly according to claim 5, **characterized in that** the evaluation unit detects and evaluates capacitance changes on the electrode surfaces (4), whereby the individual electrode surfaces (4) form an electrode of a measuring capacitor, whereby the counterelectrode of the measuring capacitor is formed by an opposite electrode surface (4) on a sensor needle (3) or by a common capacitor plate.

7. Measuring assembly according to claim 5 or 6, **characterized in that** it comprises a signal generator that supplies an electrical stimulation signal to one or more of the electrode surfaces (4) when activated.

## Revendications

1. Réseau de capteurs tridimensionnel pour la mesure de signaux électriques dans des ensembles de cellules biologiques, comprenant :
- plusieurs plaques de capteur micro-structurées (1), comprenant respectivement une section de support (2) sur laquelle sont disposées plusieurs aiguilles de capteur (3) à la façon d'un peigne, celles-ci étant ainsi espacées les unes des autres dans une première direction (X), chaque aiguille de capteur (3) possédant plusieurs surfaces d'électrode (4) réparties dans la direction longitudinale (Z) sur l'aiguille de capteur (3), lesquelles sont respectivement mises en contact avec une piste conductrice propre (6), et les pistes conductrices (6) s'étendant vers une section de mise en contact (7) par le biais de la section de support (2) ;
- plusieurs éléments d'espacement (11) fixés entre les plaques de capteur (1), de sorte qu'aussi bien les sections de support (2) que les aiguilles de capteur (3) des plaques de capteur (1) respectivement voisines sont espacées les unes des autres dans une deuxième direction (Y),
**caractérisé en ce qu'**entre les éléments d'espacement (11) et les sections de support (2) sont formés des passages (12) permettant un écoulement de fluide à travers le réseau de capteurs, entre les plaques de capteur (1) dans la direction longitudinale (Z) des aiguilles de capteur (3), **en ce que** les plaques de capteur sont constituées d'un substrat isolant, et **en ce que** la surface supérieure des plaques de capteur présente une couche de passivation biocompatible et isolante.

2. Réseau de capteurs selon la revendication 1, **caractérisé en ce que** les éléments d'espacement (11) s'étendent exclusivement entre les sections de support (2) des plaques de capteur (1) et laissent des espaces libres entre les aiguilles de capteur (3).

3. Réseau de capteurs selon la revendication 1 ou 2, **caractérisé en ce que** les pistes conductrices (6) sont pourvues d'un blindage à efficacité électromagnétique.

4. Réseau de capteurs selon l'une des revendications 1 à 3, **caractérisé en ce que** les aiguilles de capteur (3) présentent des nanostructures en forme d'ardillons sur leurs surfaces supérieures.

5. Dispositif de mesure pour la mesure d'activités électriques d'ensembles de cellules biologiques, **caractérisé en ce qu'**il comprend un réseau de capteurs selon l'une des revendications 1 à 4, lequel est raccordé à une unité d'évaluation chargée de détecter et de traiter les signaux émis par la pluralité de surfaces d'électrode (4) du réseau de capteurs, avec une résolution locale et temporelle.

6. Dispositif de mesure selon la revendication 5, **caractérisé en ce que** l'unité d'évaluation détecte et évalue des modifications de capacité au niveau des surfaces d'électrode (4), les différentes surfaces d'électrode (4) formant respectivement une électrode d'un condensateur de mesure, la contre-électrode du condensateur de mesure étant formée respectivement par une surface d'électrode opposée (4) sur une aiguille de capteur (3) ou une plaque de condensateur commune.

7. Dispositif de mesure selon la revendication 5 ou 6, **caractérisé en ce qu'**il comprend un générateur de signaux fournissant un signal de stimulation à l'une ou plusieurs des surfaces d'électrode (4) lors de l'activation.
